(19) Europäisches Patentamt
European Patent Office
Office européen des brevets

(11) EP 2 185 630 B1

(12) **EUROPÄISCHE PATENTSCHRIFT**

(45) Veröffentlichungstag und Bekanntmachung des Hinweises auf die Patenterteilung:
**10.07.2013 Patentblatt 2013/28**

(21) Anmeldenummer: **08848214.6**

(22) Anmeldetag: **07.11.2008**

(51) Int Cl.:
***C08J 3/075*** *(2006.01)* ***C08F 6/00*** *(2006.01)*
***A61L 15/60*** *(2006.01)* ***A61L 15/24*** *(2006.01)*
***C08K 5/42*** *(2006.01)* ***C08J 3/24*** *(2006.01)*
***C08F 20/06*** *(2006.01)* ***C08F 2/10*** *(2006.01)*

(86) Internationale Anmeldenummer:
**PCT/EP2008/065123**

(87) Internationale Veröffentlichungsnummer:
**WO 2009/060062 (14.05.2009 Gazette 2009/20)**

(54) **WASSERABSORBIERENDE POLYMERGEBILDE MIT VERBESSERTER FARBSTABILITÄT**

WATER-ABSORBING POLYMER STRUCTURE WITH IMPROVED COLOR STABILITY

STRUCTURES POLYMÈRES ABSORBANT L'EAU QUI PRÉSENTENT UNE MEILLEURE STABILITÉ DE COULEUR

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **08.11.2007 DE 102007053619**

(43) Veröffentlichungstag der Anmeldung:
**19.05.2010 Patentblatt 2010/20**

(73) Patentinhaber: **Evonik Degussa GmbH**
**45128 Essen (DE)**

(72) Erfinder: **WALDEN, Mirko**
**45699 Herten (DE)**

(56) Entgegenhaltungen:
**EP-A- 0 412 363**
**WO-A-2004/022609**
**WO-A-2004/084962**
**WO-A-2009/011717**
**US-B1- 6 359 049**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 185 630 B1

## Beschreibung

Die vorliegende Erfindung betrifft ein Verfahren zur Herstellung wasserabsorbierender Polymergebilde. Die Erfindung betrifft auch die durch dieses Verfahren erhältlichen wasserabsorbierenden Polymergebilde, wasserabsorbierende Polymergebilde, sowie die Verwendung wasserabsorbierender Polymergebilde.

Superabsorber sind wasserunlösliche, vernetzte Polymere, die in der Lage sind, unter Quellung und Ausbildung von Hydrogelen große Mengen an Wasser, wässrigen Flüssigkeiten, insbesondere Körperflüssigkeiten, vorzugsweise Urin oder Blut, aufzunehmen und unter Druck zurückzuhalten. Superabsorber absorbieren vorzugsweise mindestens das 100-fache ihres Eigengewichts an Wasser. Weitere Einzelheiten zu Superabsorbern sind in "Modern Superabsorbent Polymer Technology", F. L. Buchholz, A. T. Graham, Wiley-VCH, 1998" offenbart. Durch diese charakteristischen Eigenschaften sind diese wasserabsorbierenden Polymere hauptsächlich in Sanitärartikeln wie beispielsweise Babywindeln, Inkontinenzprodukten oder Damenbinden eingearbeitet.

Bei den gegenwärtig kommerziell verfügbaren Superabsorbern handelt es sich im wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind. Diese sind dadurch erhältlich, dass monomere Acrylsäure bzw. deren Salze in Gegenwart geeigneter Vernetzungsmittel radikalisch polymerisiert werden. Dabei können unterschiedliche Polymerisationsverfahren zur Anwendung gelangen, wie beispielsweise die Lösungspolymerisation, die Emulsionspolymerisation oder die Suspensionspolymerisation. Letztendlich werden durch diese unterschiedlichen Verfahren wasserabsorbierende Polymere in partikulärer Form mit einem Partikeldurchmesser in einem Bereich von 150 bis 850 μm erhalten, die dann in die Sanitärartikel eingearbeitet werden.

Derartige Superabsorber neigen jedoch dazu, sich bei längerer Lagerung zu verfärben. Zudem wird die Tendenz, dass sich ihr sauberes, frisches weiß honigbraun verfärbt, bei zunehmenden Lagerzeiten, Temperaturen und Luftfeuchtigkeiten noch beschleunigt. Unter gemäßigten klimatischen Verhältnissen, wie sie in den Vereinigten Staaten von Amerika und in Europa herrschen, ist die Verfärbungsgeschwindigkeit eines Superabsorbers allerdings so langsam, dass der Superabsorber bzw. das superabsorberhaltige Erzeugnis normalerweise schon aufgebraucht ist, ehe man eine Farbänderung mit bloßem Auge erkennen kann. In tropischen und subtropischen Klimazonen, wie in Südamerika und Südostasien, verläuft die Verfärbung des Superabsorbers jedoch so schnell, dass eine Farbänderung noch vor dem Gebrauch des Superabsorbers bzw. des superabsorberhaltigen Erzeugnisses eintritt.

Verantwortlich für die Verfärbung des Superabsorbers sind in erster Linie die bei der radikalischen Polymerisation eingesetzten und im Polymerisat verbleibenden Initiatoren, wie beispielsweise Ascorbinsäure und Natriumperoxodisulfat, sowie Inhibitoren, die in der Regel in der eingesetzten Acrylsäure zum Zwecke der Verhinderung einer spontan erfolgenden Polymerisation enthalten sind, wie beispielsweise der Monomethylether des Hydroquinons (MEHQ).

Zur Verbesserung der Farbstabilität von Superabsorbern schlägt daher WO-A-2004/084962 vor, der Monomerlösung anstelle der üblichen Initiatorsysteme beinhaltend Persulfate ein Sulfinat oder ein Salz eines Sulfinates zuzusetzen. Konkret werden in der WO-A-2004/084962 die von der Firma Brüggemann Chcmical, Heilbronn, Deutschland, kommerziell erhältlichen Produkte BRUGGOLITE®FF6 und BRUGGOLITE®FF7 der Monomerlösung als Initiatorsystem zugesetzt, wobei es sich bei dem Produkt BRUGGOLITE®FF6 um eine Mischung aus 2-Hydroxy-2-Sulfinatoessigsäure-Dinatriumsalz, Natriumsulfit und 2-Hydroxy-2-Sulfonatoessigsäure-Dinatriumsalz, während BRUGGOLITE®FF7 reine 2-Hydroxy-2-Sulfinatoessigsäure enthält.

Das in der WO-A-2004/084962 beschriebene Verfahren zur Verbesserung der Farbstabilität weist jedoch den Nachteil auf, dass die dort als Initiatorsystem eingesetzten Sulfinate bzw. dort als Initiatorsystem eingesetzten Mischungen aus Sulfinaten, Sulfonaten und Sulfiten im Vergleich zu den herkömmlich eingesetzten Peroxiden unter bestimmten Bedingungen schlechtere Initiatoren der radikalischen Polymerisation sind. Die verbesserte Farbstabilität geht daher auch zu Lasten der Polymerisationsreaktion und mithin auch zu Lasten der Absorptionseigenschaften der bei dieser Polymerisationsreaktion erhaltenen Polymerisate.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, ein Verfahren zur Herstellung wasserabsorbierende Polymergebilde mit verbesserter Farbstabilität anzugeben, wobei die durch dieses Verfahren erhältlichen Polymerisate auch durch eine gute Geruchsstabilität gekennzeichnet sind.

Auch lag der vorliegenden Erfindung die Aufgabe zugrunde, ein Verfahren zur Herstellung wasserabsorbierende Polymergebilde mit verbesserter Farbstabilität anzugeben, bei dem auf herkömmliche Initiatorsysteme, die sich als durchaus vorteilhaft erwiesen haben, zurückgegriffen werden kann.

Darüber hinaus lag der vorliegenden Erfindung die Aufgabe zugrunde, wasserabsorbierende Polymergebilde mit verbesserter Farbstabilität bereitzustellen, die trotz ihrer verbesserten Farbstabilität den aus dem Stand der Technik bekannten Polymerisaten hinsichtlich ihrer Absorptionseigenschaften nicht unterlegen sind.

Einen Beitrag zur Lösung der eingangs genannten Aufgaben leistet ein Verfahren zur Herstellung wasserabsorbierender Polymergebilde, gemäβ jedem einzelnen der Ansprüche 1-5.

Die bevorzugten Polymergebilde sind Fasern, Schäume oder Teilchen, wobei Fasern und Teilchen bevorzugt und Teilchen besonders bevorzugt sind.

Bevorzugte Polymerfasern sind so dimensioniert, dass sie in oder als Garne für Textilien und auch direkt in Textilien eingcarbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Bevorzugte Polymerteilchen sind so dimensioniert, dass sie eine mittlere Teilchengröße gemäß ERT 420.2-02 im Bereich von 10 bis 3000 μm, vorzugsweise 20 bis 2000 μm und besonders bevorzugt 150 bis 850 μm aufweisen. Dabei ist es insbesondere bevorzugt, dass der Anteil der Polymerteilchen mit einer Partikelgröße in einem Bereich von 300 bis 600 μm mindestens 30 Gew.-%, besonders bevorzugt mindestens 40 Gew.-% und am meisten bevorzugt mindestens 50 Gew.-%, bezogen auf das Gesamtgewicht der nachvemetzten, wasserabsorbierenden Polymerteilchen, beträgt.

Gemäß einer besonderen Ausführungsform des erfindungsgemäßen Verfahrens sind die Polymerteilchen so dimensioniert, dass sie eine mittlere Teilchengröße gemäß ERT 420.2-02 im Bereich von 150 bis 710 μm, besonders bevorzugt im Bereich von 150 bis 600 μm aufweisen.

Im Verfahrensschritt i) des erfindungsgemäßen Verfahrens wird zunächst eine wässrige Monomerlösung bereitgestellt.

Die monoethylenisch ungesättigten, säuregruppen-tragenden Monomere (α1) können teilweise oder vollständig, bevorzugt teilweise neutralisiert sein. Vorzugsweise sind die monoethylenisch ungesättigten, säuregruppen-tragenden Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem durch das erfindungsgemäße Verfahren erhältlichen wasserabsorbierenden Polymergebilde die freien Säuregruppen überwiegen, so dass dieses Polymergebilde einen im sauren Bereich liegenden pH-Wert aufweist. Dieses saure wasserabsorbierende Polymergebilde kann durch ein Polymergebilde mit freien basischen Gruppen, vorzugsweise Amingruppen, das im Vergleich zu dem sauren Polymergebilde basisch ist, mindestens teilweise neutralisiert werden. Diese Polymergebilde werden in der Literatur als "Mixed-Bed Ion-Exchange Absorbent Polymers" (MBIEA-Polymere) bezeichnet und sind unter anderem in der WO 99/34843 A1 offenbart. In der Regel stellen MBIEA-Polymere eine Zusammensetzung dar, die zum einen basische Polymergebilde, die in der Lage sind, Anionen auszutauschen, und andererseits ein im Vergleich zu dem basischen Polymergebilde saures Polymergebilde, das in der Lage ist, Kationen auszutauschen, beinhalten. Das basische Polymergebilde weist basische Gruppen auf und wird typischerweise durch die Polymerisation von Monomeren (α1) erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte monoethylenisch ungesättigte, säuregruppen-tragende Monomere (α1) sind vorzugsweise diejenigen Verbindungen, die in der WO 2004/037903 A2, als ethylenisch ungesättigte, säuregruppenhaltige Monomere (α1) genannt werden. Besonders bevorzugte monoethylenisch ungesättigte, säuregruppen-tragende Monomere (α1) sind Acrylsäure und Methacrylsäure, wobei Acrylsäure am meisten bevorzugt ist.

Als mit den Monomeren (α1) copolymerisierbare, monoethylenisch ungesättigte Monomere (α2) können Acrylamide, Methacrylamide oder Vinylamide eingesetzt werden.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth) acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Weiterhin können als mit den Monomeren (α1) copolymerisierbare, monoethylenisch ungesättigte Monomere (α2) wasserlösliche Monomere eingesetzt werden. In diesem Zusammenhang sind insbesondere Alkoxypolyalkylenoxid (meth)acrylate wie Methoxypolyethylenglykol(meth)acrylate bevorzugt.

Als monoethylenisch ungesättigte, mit den Monomeren (α1) copolymerisierbare Monomere (α2) sind weiterhin in Wasser dispergierbare Monomere denkbar. Als in Wasser dispergierbare Monomere sind Acrylsäureester und Methacrylsäureester, wie Methyl(meth)acrylat, Ethyl(meth)acrylat, Propyl(mcth)acrylat oder Butyl(meth)acrylat bevorzugt.

Die monoethylenisch ungesättigten, mit ($\alpha$1) copolymerisierbaren Monomere ($\alpha$2) können auch Methylpolyethylenglykolallylether, Vinylacetat, Styrol und Isobutylen umfassen.

Als Vernetzer ($\alpha$3) werden vorzugsweise diejenigen Verbindungen eingesetzt, die in der WO 2004/037903 A2 als Vernetzer ($\alpha$3) genannt werden. Unter diesen Vernetzern sind wasserlösliche Vernetzer besonders bevorzugt. Am meisten bevorzugt sind dabei N,N'-Methylenbisacrylamid, Polyethylenglykol-di(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Neben den Monomeren ($\alpha$1) und gegebenenfalls ($\alpha$2) sowie gegebenenfalls dem Vernetzer ($\alpha$3) kann die Monomerlösung auch wasserlösliche Polymere ($\alpha$4) beinhalten. Bevorzugte wasserlösliche Polymere ($\alpha$4) umfassend teil- oder vollverseiften Polyvinylalkohol, Polyvinylpyrrolidon, Stärke oder Stärkederivate, Polyglykole oder Polyacrylsäure. Das Molekulargewicht dieser Polymere ist unkritisch, solange sie wasserlöslich sind. Bevorzugte wasserlösliche Polymere ($\alpha$4) sind Stärke oder Stärkederivate oder Polyvinylalkohol. Die wasserlöslichen Polymere ($\alpha$4), vorzugsweise synthetische wie Polyvinylalkohol, können nicht nur als Pfropfgrundlage für die zu polymerisierenden Monomeren dienen. Denkbar ist auch, diese wasserlöslichen Polymere erst nach der Polymerisation mit dem Polymergel oder dem bereits getrockneten, wasserabsorbierenden Polymergel zu vermischen.

Weiterhin kann die Monomerlösung auch Hilfsmittel ($\alpha$5) enthalten, wobei zu diesen Hilfsmitteln insbesondere die für die Polymerisation gegebenenfalls erforderlichen Initiatoren, Komplexbildner, wie beispielsweise EDTA, aber insbesondere auch thermoplastische Polymere oder Dispersionen beinhaltend thermoplastische Polymere gehören.

Als Lösungsmittel für die Monomerlösung kommen Wasser, organische Lösungsmittel oder Gemische aus Wasser und organischen Lösungsmitteln in Betracht, wobei die Wahl des Lösungsmittels insbesondere auch von der Art und Weise der Polymerisation abhängt.

Die relative Menge an Monomeren ($\alpha$1) und ($\alpha$2) sowie an Vemetzem ($\alpha$3) und wasserlöslichen Polymeren ($\alpha$4) und Hilfsmittel ($\alpha$5) in der Monomerlösung wird vorzugsweise so gewählt, dass das im Verfahrensschritt iv) nach dem Trocknen erhaltene wasserabsorbierende Polymergebilde

- zu 20-99,999 Gew.-%, bevorzugt zu 55-98,99 Gew.-% und besonders bevorzugt zu 70-98,79 Gew.-% auf den Monomeren ($\alpha$1),
- zu 0-80 Gew.-%, vorzugsweise zu 0-44,99 Gew.-% und besonders bevorzugt zu 0,1-44,89 Gew.-% auf den Monomeren ($\alpha$2),
- zu 0-5 Gew.-%, vorzugsweise zu 0,001-3 Gew.-% und besonders bevorzugt zu 0,01-2,5 Gew.-% auf den Vernetzern ($\alpha$3),
- zu 0-30 Gew.-%, vorzugsweise zu 0-5 Gew.-% und besonders bevorzugt zu 0,1-5 Gew.-% auf den wasserlöslichen Polymeren ($\alpha$4),
- zu 0-20 Gew.-%, vorzugsweise zu 0-10 Gew.-% und besonders bevorzugt zu 0,1-8 Gew.-% auf den Hilfsmitteln ($\alpha$5), und
- zu 0,5-25 Gew.-%, vorzugsweise zu 1-10 Gew.-% und besonders bevorzugt zu 3-7 Gew.-% auf Wasser ($\alpha$6)

basiert, wobei die Summe der Gewichtsmengen ($\alpha$1) bis ($\alpha$6) 100 Gew.-% beträgt.

Optimale Werte für die Konzentration insbesondere der Monomere, Vemetzer und wasserlöslichen Polymere in der Monomerlösung können durch einfache Vorversuche ermittelt oder aber auch dem Stand der Technik, insbesondere den Druckschriften US 4,286,082, DE 27 06 135 A1, US 4,076,663, DE 35 03 458 A1, DE 40 20 780 C1, DE 42 44 548 A1, DE 43 33 056 A1 und DE 44 18 818 A1 entnommen werden.

Im Verfahrensschritt ii) des erfindungsgemäßen Verfahrens wird die im Verfahrensschritt i) erhaltene wässrigen Monomerlösung unter Erhalt eines Polymergels radikalisch polymerisiert, wobei grundsätzlich alle dem Fachmann bekannten Polymerisationsverfahren in Betracht kommen können. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135 A1, US 4,076,663, DE 35 03 458 A1, DE 40 20 780 C1, DE 42 44 548 A1, DE 43 33 056 A1, DE 44 18 818 A1.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in

Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt. In diesem Zusammenhang ist es insbesondere bevorzugt, wenn der Monomerlösung mehr als 300 ppm eines Persulfates, insbesondere mehr als 300 ppm Natriumperoxodisulfat, bezogen auf das Gesamtgewicht der Monomerlösung, zugesetzt werden.

Auch die inverse Suspensions- und Emulsionspolymerisation kann im erfindungsgemäßen Verfahren angewendet werden. Gemäß diesen Prozessen wird eine wässrige, teilneutralisierte Lösung der Monomeren (α1) und (α2), gegebenenfalls beinhaltend die wasserlöslichen Polymere (α4) und Hilfsmittel (α5), mit Hilfe von Schutzkolloiden und/ oder Emulgatoren in einem hydrophoben, organischen Lösungsmittel dispergiert und durch Radikalinitiatoren die Polymerisation gestartet. Die Vernetzer (α3) sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers (α3) und/oder durch Reaktion geeigneter Vemetzer mit funktionellen Gruppen des Polymeren während der Polymerisationsschritte erfolgen. Die Verfahren sind beispielsweise in den Veröffentlichungen US 4,340,706, DE 37 13 601 A1, DE 28 40 010 A1 und WO 96/05234 A1 beschrieben.

Im Verfahrensschritt iii) des erfindungsgemäßen Verfahrens wird das im Verfahrensschritt ii) erhaltene Polymergel gegebenenfalls zerkleinert, wobei dieses Zerkleinem insbesondere dann erfolgt, wenn die Polymerisation mittels einer Lösungspolymerisation durchgeführt wird. Das Zerkleinern kann durch dem Fachmann bekannte Zerkleinerungsvorrichtungen, wie etwa einem Fleischwolf, erfolgen.

Im Verfahrensschritt iv) des erfindungsgemäßen Verfahrens wird das gegebenenfalls zuvor zerkleinerte Polymergel getrocknet. Die Trocknung des Polymergels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Polymergels im Verfahrensschritt iv) bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Im Verfahrensschritt v) des erfindungsgemäßen Verfahrens können die im Verfahrensschritt iv) erhaltenen, wasserabsorbierenden Polymergebilde insbesondere dann, wenn sie durch Lösungspolymerisation erhalten wurden, noch zermahlen und auf die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle, während das Absieben beispielsweise durch Verwendung von Sieben mit geeigneter Maschenweite erfolgen kann.

Im Verfahrensschritt vi) des erfindungsgemäßen Verfahrens werden die gegebenenfalls zermahlenen und abgesiebten wasserabsorbierenden Polymergebilde o-berflächennachvemetzt. Zur Oberflächennachvernetzung werden die getrockneten und gegebenenfalls zermahlenen und abgesiebten wasserabsorbierenden Polymcrgebilde aus dem Verfahrensschritt iv) oder v) aber das noch nicht getrocknete, jedoch vorzugsweise bereits zerkleinerte Polymergel aus dem Verfahrensschritt ii) oder iii) mit einem vorzugsweise organischen, chemischen Oberflächennachvernetzcr in Kontakt gebracht wird. Dabei wird der Nachvernetzer insbesondere dann, wenn er unter den Nachvemetzungsbedingungen nicht flüssig ist, vorzugsweise in Form eines Fluids $F_1$ umfassend den Nachvemetzer sowie ein Lösungsmittel mit den wasserabsorbierenden Polymergebilde bzw. dem Polymergel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvemetzer in dem Fluid $F_1$ in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids $F_1$, enthalten ist.

Das in Kontakt bringen des wasserabsorbierenden Polymergebildes bzw. des gegebenenfalls zerkleinerten Polymergels mit dem Nachvernetzer bzw. mit dem Fluid $F_1$ beinhaltend den Nachvernetzer erfolgt im erfindungsgemäßen Verfahren vorzugsweise durch gutes Vermischen des Nachvernetzers bzw. des Fluid $F_1$ mit dem Polymergebilde bzw. dem Polymergel.

Geeignete Mischaggregate zum Aufbringen des Nachvernetzers bzw. des Fluids $F_1$ sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde bzw. das Polymergel wird in dem erfindungsgemäßen Verfahren bei der Nachvernetzung vorzugsweise mit höchstens 20 Gcw. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymergebilden in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen Polymergebilde mit dem Nachvernetzer bzw. mit dem Fluid $F_1$ in Kontakt gebracht werden.

Als Nachvemetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die Polymergebilde bzw. die Polymergele mit dem Nachvernetzer bzw. mit dem Fluid $F_1$ beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt (=Nachvernetzung) und, sofern Polymergel eingesetzt werden, diese zugleich auch getrocknet werden. Die Zeitdauer der Wärmebehandlung wird durch die Gefahr, dass das gewünschte Eigenschaftsprofil der Polymergebilde infolge von Hitzeeinwirkung zerstört wird, begrenzt.

Gemäß dem erfindungsgemäßen Verfahren wird

I) dem wasserabsorbierenden Polymergebilde nach Durchführung des Verfahrensschrittes iv),

II) dem wasserabsorbierenden Polymergebilde nach Durchführung des Verfahrensschrittes v),

III) dem wasserabsorbierenden Polymergebilde nach Durchführung des Verfahrensschrittes vi), ein Reduktionsmittel beinhaltend ein Sulfonat, ein Salz eines Sulfonates oder eine Mischung aus einem Sulfonat und einem Salz eines Sulfonates zugesetzt, entsprechend Anspruch 1.

Jeder der vorstehend genannten Alternativen I), II), III), stellt eine bevorzugte Ausführungsform des erfindungsgemäßen Verfahrens dar, wobei das Reduktionsmittel auch zu mehren verschiedenen Zeitpunkten zugesetzt werden kann.

Gemäß einer ersten und besonders bevorzugten Variante des erfindungsgemäßen Verfahrens jedoch wird das Reduktionsmittel nach Durchführung des Verfahrensschrittes iv) oder aber nach Durchführung des Verfahrensschrittes v), jedoch vor Durchführung des Verfahrensschrittes vi) zugesetzt, wobei es sich insbesondere als Vorteilhaft erweisen kann, das Reduktionsmittel dem Fluid $F_1$ beinhaltend den Nachvernetzer und das Lösungsmittel zuzusetzen. Denkbar ist aber auch, dass Reduktionsmittel in Form eines separaten Fluids $F_2$ beinhaltend das Reduktionsmittel und ein Lösungsmittel, beispielsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel, wobei Wasser als Lösungsmittel im Fluid $F_2$ besonders ist, vor oder, während oder nach dem Applizieren des Fluids $F_1$ zuzusetzen. Nachdem auf diese Weise beide Komponenten (Reduktionsmittel und Nachvernetzer) mit dem wasserabsorbierenden Polymergebilde in Kontakt gebracht worden sind, erfolgt die Nachvernetzung gemäß Verfahrensschritt vi) des erfindungsgemäßen Verfahrens durch das Erhitzen der so erhaltenen Mischung auf die im Zusammenhang mit der Oberflächennachvernetzung vorstehend genannten Temperaturbereiche.

Gemäß einer zweiten besonders bevorzugten Variante des erfindungsgemäßen Verfahrens wird das Reduktionsmittel nach Durchführung des Verfahrensschrittes vi) mit dem wasserabsorbierenden Polymergebilde in Kontakt gebracht. Dazu kann das Reduktionsmittel beispielsweise in Form des vorstehend beschriebenen Fluids $F_2$ mit dem bereits oberflächennachvernetzten, wasserabsorbierenden Polymergebilde vermischt werden.

Im Zusammenhang mit dem eingesetzten Reduktionsmittel sind weniger als 10 Gew.-%, besonders bevorzugt weniger als 5 Gew.-%, noch mehr bevorzugt weniger als 1 Gew.-%, jeweils bezogen auf das Gesamtgewicht des Reduktionsmittels, von Sulfonaten und von Sulfonat-Salzen verschiedenen Schwefel-Verbindungen, insbesondere weniger als 10 Gew.-%, besonders bevorzugt weniger als 5 Gew.-%, noch mehr bevorzugt weniger als 1 Gew.-% Sulfite,

Bisulfite, Sulfinate, insbesondere 2-Hydroxy-2-Sulfinatoessigsäure, oder Salze, insbesondere Natriumsalze, dieser Schwefelverbindungen, am meisten bevorzugt Natriumsulfit und das Dinatriumsalz der 2-Hydroxy-2-Sulfinatoessigsäure, beinhaltet. Es werden in dem erfindungsgemäßen Verfahren insbesondere weniger als 1.000 ppm, besonders bevorzugt weniger als 500 ppm und noch mehr bevorzugt weniger als 100 ppm, jeweils bezogen auf den Feststoffgehalt des wasserabsorbierenden Polymergebildes, am meisten bevorzugt jedoch überhaupt keine von Sulfonaten und von Sulfonat-Salzen verschiedenen Schwefel-Verbindungen zur Behandlung des nach Durchführung des Verfahrensschrittes iv) erhaltenen, wasserabsorbierenden Polymergebildes, des nach Durchführung des Verfahrensschrittes v) erhaltenen, wasserabsorbierenden Polymergebildes oder des nach Durchführung des Verfahrensschrittes vi) erhaltenen, wasserabsorbierenden Polymergebilde eingesetzt. Das ein-gesetzte Reduktionsmittel besteht zu mindestens 90 Gew.-%, besonders bevorzugt zu mindestens 95 Gew.-% und am meisten bevorzugt zu mindestens 99 Gew.-%, jeweils bezogen auf das Gesamtgewicht des eingesetzten Reduktionsmittels, aus Sulfonaten, aus Salzen von Sulfonaten oder aus einer Mischung aus Sulfonaten und Salzen von Sulfonaten entsprechend Anspruch 1.

Die Formulierung *"beinhaltend weniger als 10 Gew.-%, bevorzugt weniger als 5 Gew.-%, mehr bevorzugt weniger als* 1 *Gew.-% Sulfite, Bisulfite, Sulfinate oder Salze dieser Schwefelverbindungen"* ist dabei so zu verstehen, dass die Gesamtmenge an Sulfiten, Bisulfiten, Sulfinaten oder Salzen dieser Schwefel-verbindungen weniger als 10 Gew.-%, bevorzugt weniger als 5 Gew.-%, mehr bevorzugt weniger als 1 Gew.-% beträgt.

Als weitere, im Reduktionsmittel enthaltene Sulfonate bzw. Salz des Sulfonates können genannt werden: die Struktur I

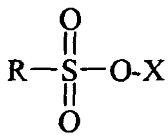

Struktur I

, in der X ein Wasserstoffatom, ein Ammonium-Kation oder ein Alkalimetall-Kation und R

- ein gesättigter oder ungesättigter, aliphatischer oder aromatischer $C_1$-$C_{20}$-Kohlenwasserstoff-Rest, besonders bevorzugt ein gesättigter oder ungesättigter, aliphatischer oder aromatischer $C_2$-$C_{15}$-Kohlenwasserstoff-Rest und am meisten bevorzugt ein gesättigter oder ungesättigter, aliphatischer oder aromatischer $C_3$-$C_{10}$-Kohlenwasserstoff Rest,

- ein Rest der Struktur YOOC-$R^1$-, in der Y ein Wasserstaffatom, ein Ammonium-Kation oder ein einwertiges Metall-Kation, vorzugsweise ein Alkalimetall-Kation, und $R^1$ ein $C_1$-$C_7$-Alkylen-Rest, bevorzugt ein $C_1$-$C_5$-Alkylen-Rest und am meisten bevorzugt ein $C_1$-$C_3$-Alkylen-Rest ist, wobei die YOOC-Funktion gegebenenfalls auch mit 2 bis 50 Alkylenoxid-Einheiten, besonders bevorzugt mit 4 bis 20 Alkylenoxid-Einheiten. besonders bevorzugt Ethylenoxid- oder Propylenoxid-Einheiten, alkoxyliert sein kann,

- ein Rest der Struktur ZOOC-$R^2$-, in der Z ein Wasserstoffatom, ein Ammonium-Kation oder ein einwertiges Metall-Kation, vorzugsweise ein Alkalimetall-Kation, und $R^2$ ein $C_1$-$C_7$-Alkylen-Rest, bevorzugt ein $C_1$-$C_5$-Alkylen-Rest um am meisten bevorzugt ein $C_1$-$C_3$-Alkylen-Rest ist, der an mindestens einem der Kohlenstoffatome eine Hydroxylgruppe trägt, wobei die ZOOC-Funktion gegebenenfalls auch mit 2 bis 50 Alkylenoxid-Einheiten, besonders bevorzugt mit 4 bis 20 Alkylenoxid-Einheiten. besonders bevorzugt Ethylenoxid- oder Propylenoxid-Einheiten, alkoxyliert sein kann,

- ein Rest der Struktur R'R"N-$R^3$-, in der $R^3$ ein $C_1$-$C_9$-Alkylen-Rest, bevorzugt ein $C_2$-$C_8$-Alkylen-Rest und am meisten bevorzugt ein $C_3$-$C_7$-Alkylen-Rest ist, und die Reste R' und R" ein Wasserstoffatom, ein $C_1$-$C_5$-Alkyl-Rest oder ein Rest der Struktur R"'-CO-, in der R"' ein gesättigter oder ungesättigter $C_1$-$C_5$-Kohlenwasserstoff ist, sein können, oder

- ein Rest der Struktur $R^4$-$[OCH_2CH_2]_n$-O-$R^5$- oder ein Rest der Struktur $R^4$-$[OCH_2CHCH_3]_n$-O-$R^5$-, in denen $R^4$ ein $C_1$-$C_{10}$-Alkyl-Rest oder ein $C_1$-$C_9$-Acyl-Rest, n eine ganze Zahl in einem Bereich von 2 bis 50, besonders bevorzugt in einem Bereich von 4 bis 20 und $R^5$ ein $C_1$-$C_9$-Alkylen-Rest ist.

Beispiele weiterer Sulfonate umfassen insbesondere Vinylsulfonsäure, Allylsulfonsäure, Vinyltoluolsulfonsäure, Styrolsulfonsäure, Sulfoethylacrylat, Sulfoethylmethacrylate, Sulfopropylacrylate, Sulfopropylmethacrylate, 2-Vinyl-4-ethylbenzolsulfonsäure, 2-Allylbenzolsulfonsäure, 1-Phenylethylenesulfonsäure, 2-Hydroxy-3-methacryloxypropylsulfonsäure, 2-Acrylamid-2-methylpropansulfonsäure, para-Toluolsulfonsäure, , die entsprechenden Salzen der vorstehend genannten Verbindungen sowie Mischungen aus mindestens zwei der vorstehend genannten Verbindungen.

Erfindungsgemäß ist der Einsatz von 2-Hydroxy-2-Sulfonatoessigsäure, insbesondere des Dinatriumsalzes der 2-Hydroxy-2-Sulfonatoessigsäure bzw. der Einsatz eines Reduktionsmittels, welches zu mindestens 90 Gew.-%, mehr bevorzugt zu mindestens 95 Gew.-% und am meisten bevorzugt zu mindestens 99 Gew.-% aus 2-Hydroxy-2-Sulfonatoessigsäure, dem Dinatriumsalz der 2-Hydroxy-2-Sulfonatoessigsäure oder Mischungen der 2-Hydroxy-2-Sulfonatoessigsäure und des Dinatriumsalzes der 2-Hydroxy-2-Sulfonatoessigsäure besteht.

Weiterhin ist es erfindungsgemäß bevorzugt, dass das Reduktionsmittel in einer Menge in einem Bereich von 0,001 bis 10 Gew.-%, besonders bevorzugt in einem Bereich von 0,005 bis 5 Gew.-% und am meisten bevorzugt in einer Menge in einem Bereich von 0,01 bis 1 Gew.-%, jeweils bezogen auf den Feststoffgehalt des wasserabsorbierenden Polymergebildes, eingesetzt wird.

Neben der im Verfahrensschritt vi) erfolgenden Oberflächenmodifizierung der wasserabsorbierenden Polymergebilde mittels Oberflächennachvernetzung können die wasserabsorbierenden Polymergebilde auch weiteren Oberflächenmodifizierungen unterworfen werden, welche grundsätzlich vor, während oder nach der Oberflächenmodifizierung erfolgen können.

Als bevorzugte Modifizierungsmaßnahme sei hier das in Kontakt bringen des Außenbereiches der Polymergebilde mit einer Verbindung enthaltend ein zwei- oder höherwertiges Metall-Ion, besonders bevorzugt ein $Al^{3+}$-Ion, vor, während oder nach der Nachvernetzung (also vor, während oder nach dem Verfahrensschritt vi)) zu nennen. Dabei ist es bevorzugt, dass die Verbindung enthaltend ein zwei- oder höherwertiges Metall-Ion in einer Menge in einem Bereich von 0,01 bis 30 Gew. - %, besonders bevorzugt in einer Menge in einem Bereich von 0,1 bis 20 Gew. -% und darüber hinaus bevorzugt in einer Menge in einem Bereich von 0,3 bis 5 Gew. -%, jeweils bezogen auf das Gewicht der wasserabsorbierenden Polymergebilde, mit den wasserabsorbierenden Polymergebilden in Kontakt gebracht wird.

Das in Kontakt bringen des Außenbereiches der wasserabsorbierenden Polymergebilde mit der Verbindung enthaltend ein zwei- oder höherwertiges Metall-Ion erfolgt vorzugsweise dadurch, dass das wasserabsorbierende Polymergebilde mit der Verbindung unter trockenen Bedingungen vermischt wird oder aber dadurch, dass die wasserabsorbierenden Polymergebilde mit einem Fluid $F_3$ umfassend ein Lösemittel, vorzugsweise Wasser, mit Wasser mischbare organische Lösemittel wie etwa Methanol oder Ethanol oder Mischungen aus mindestens zwei davon, sowie die Verbindung enthaltend ein zwei- oder höherwertiges Metall-Ion in Kontakt gebracht werden, wobei das in Kontakt bringen vorzugsweise durch Besprühen der wasserabsorbierenden Polymerteilchen mit dem Fluid $F_3$ und Vermischen erfolgt.

Als Verbindung enthaltend ein zwei- oder höherwertiges Metall-Ion kommen insbesondere Salze, besonders bevorzugt wasserlösliche Salze von zwei- oder höherwertigen Metallen, insbesondere Salz von Erdalkalimetall-Ionen, wie etwa Calcium- oder Magnesiumsalze, Salze des Aluminiums, des Chroms, des Kupfers, des Eisens, des Zinks oder Mischsalze dieser Kationen in Betracht, wobei Salze des Aluminiums besonders bevorzugt sind.

Vorzugsweise ist dabei die Verbindung enthaltend ein zwei- oder höherwertiges Metall-Ion ohne Berücksichtigung von Kristallwasser in einer Menge in einem Bereich von 0,1 bis 50 Gew. -%, besonders bevorzugt in einer Menge in einem Bereich von 1 bis 30 Gew.-%, jeweils bezogen auf das Gesamtgewicht des Fluids $F_3$, in dem Fluid $F_3$ enthalten. Es ist weiterhin bevorzugt, dass das Fluid $F_3$ in einer Menge in einem Bereich von 0,01 bis 15 Gew.-%, besonders bevorzugt in einer Menge in einem Bereich von 0,05 bis 6 Gew.-%, jeweils bezogen auf das Gewicht der Polymergebilde, mit den Polymergebilden in Kontakt gebracht wird.

Besonders bevorzugte Verbindung enthaltend ein zwei- oder höherwertiges Metall-Ion sind $Al^{3+}$-Ionen enthaltenden Verbindungen, wobei unter diesen wiederum $AlCl_3 \times 6H_2O$, $NaAl(SO_4)_2 \times 12\ H_2O$, $KAl(SO_4)_2 \times 12\ H_2O$, $Al_2(SO_4)_3 \times 14\text{-}18\ H_2O$ oder Aluminiumlactat, wobei Aluminiumlactat und Aluminiumsulfat bzw. Hydrate des Aluminiumsulfats besonders bevorzugt sind. Auch Mischungen dieser Aluminiumverbindungen, insbesondere Mischungen aus Aluminiumlactat und Aluminiumsulfat können eingesetzt werden. Weiterhin bevorzugte Verbindungen enthaltend ein zwei- oder höherwertiges Metall-Ion sind insbesondere Mischungen aus einem Salz beinhaltend ein Alkalimetall-Kation, besonders bevorzugt $Na^+$, und einer deprotonierten, organischen Säure, besonders bevorzugt dem Lactat-Anion, und der vorstehend beschriebenen Verbindung enthaltend ein zwei-oder höherwertiges Metall-Ion, wobei insbesondere Mischungen aus Natriumlactat und Aluminiumsulfat sowie Mischungen aus Natriumlactat und Aluminiumlactat besonders bevorzugt sind.

Gemäß einer besonderen Ausführungsform des erfindungsgemäßen Verfahrens erfolgt die Oberflächenmodifzierung der wasserabsorbierenden Polymergebilde mit der Verbindung enthaltend ein zwei- oder höherwertiges Metall-Ion vor der Durchführung des Verfahrensschrittes vi), in dem die nach Durchführung des Verfahrensschrittes iv) oder nach Durchführung des Verfahrensschrittes v) erhaltenen, wasserabsorbierenden Polymergebilde mit einem Fluid beinhaltend den Nachvernetzer, das Reduktionsmittel und die Verbindung enthaltend ein zwei-oder höherwertiges Metall-Ion in Kontakt gebracht und auf die im Zusammenhang mit der Oberflächennachvemetzung genannten Temperaturen erhitzt werden.

Als weitere Oberflächenmodifizierungsmaßnahme, neben der Oberflächennachvernetzung und der Behandlung mit einer Verbindung enthaltend ein zwei- oder höherwertiges Metall-Ion sei an dieser Stelle auch das in Kontakt

bringen der wasserabsorbierenden Polymergebilde mit anorganischen Partikeln, beispielsweise mit feinteiligem Siliziumdioxid, welches vorzugsweise in wässriger Suspension appliziert wird, oder mit Kieselsäuresol erwähnt. Denkbar ist weiterhin die Beschichtung der wasserabsorbierenden Polymergebilde mit sogenannten "anti-caking"-Mitteln, mit Fließhilfsmitteln wie etwa Polyethylenglykolen, mit thermoplastischen Polymeren, wie dies beispielsweise in der DE-A-103 34 286 beschrieben ist, oder mit geruchsbindenen Mitteln, wie etwa Cyclodextrinen oder Zeolithen.

Einen Beitrag zur Lösung der eingangs genannten Aufgaben leistet auch ein wasserabsorbierendes Polymergebilde, welches erhältlich ist durch das vorstehend beschriebene, erfindungsgemäße Verfahren. Dabei ist es besonders bevorzugt, dass das erfindungsgemäße, wasserabsorbierende Polymergebilde zu mindestens 50 Gew.-%, vorzugsweise zu mindestens 70 Gew.-% und darüber hinaus bevorzugt zu mindestens 90 Gew.-%, jeweils bezogen auf das Gewicht der wasserabsorbierenden Polymergebilde, aus carboxylatgruppen-tragenden Monomeren besteht. Es ist erfindungsgemäß weiterhin bevorzugt, dass das erfindungsgemäße, wasserabsorbierende Polymergebilde zu mindestens 50 Gew.-%, vorzugsweise zu mindestens 70 Gew.-%, jeweils bezogen auf das Gewicht der wasserabsorbierenden Polymergebilde, auf polymerisierter Acrylsäure basiert, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Einen Beitrag zur Lösung der eingangs genannten Aufgaben leistet weiterhin ein wasserabsorbierendes Polymergebilde entsprechend Anspruch 7, beinhaltend 10 bis 100.000 ppm, besonders bevorzugt 50 bis 50.000 ppm und am meisten bevorzugt 100 bis 10.000 ppm, jeweils bezogen auf den Feststoffgehalt des wasserabsorbierenden Polymergebildes, eines nicht polymerisierten Sulfonates, eines nicht polymerisierten Salzes eines Sulfonates oder einer Mischung aus einem nicht polymerisierten Sulfonat und einem nicht polymerisierten Salz eines Sulfonates, sowie weniger als 1.000 ppm, besonders bevorzugt weniger als 500 ppm, noch mehr bevorzugt weniger als 100 ppm, jeweils bezogen auf den Feststoffgehalt des wasserabsorbierenden Polymergebildes, nicht polymerisierte Sulfite, nicht polymerisierte Bisulfite, nicht polymerisierte Sulfinate oder nicht polymerisierten Salze dieser Verbindungen. Am meisten bevorzugt enthält das wasserabsorbierende Polymergebilde keine nachweisbaren Mengen an nicht polymerisierten Bisulfiten, nicht polymerisierte Sulfinaten oder nicht polymerisierten Salzen dieser Verbindungen, wobei auch hier die Formulierung *"beinhaltend weniger als 1.000 ppm nicht polymerisierte Sulfite, nicht polymerisierte Bisulfite, nicht polymerisierte Sulfinate oder nicht polymerisierten Salze dieser Verbindungen"* so zu verstehen, dass die Gesamtmenge an Sulfiten, Bisulfiten, Sulfinaten oder Salzen dieser Schwefelverbindungen weniger als 1.000 ppm, besonders bevorzugt weniger als 500 ppm, noch mehr bevorzugt weniger als 100 ppm beträgt.

Einen weiteren Beitrag zur Lösung der eingangs genannten Aufgaben leistet insbesondere ein wasserabsorbierendes Polymergebilde entsprechend Anspruch 8, dessen Oberfläche mit 0,001 bis 10 Gew.-%, besonders bevorzugt mit 0,001 bis 5 Gew.-% und am meisten bevorzugt mit 0,01 bis 1 Gew.-%, jeweils bezogen auf den Feststoffgehalt des wasserabsorbierenden Polymergebildes, eines Reduktionsmittels beinhaltend ein Sulfonat, ein Salz eines Sulfonates oder eine Mischung aus einem Sulfonat und einem Salz eines Sulfonates, in Kontakt gebracht worden ist.

In diesem Zusammenhang ist es erforderlich, dass das Reduktionsmittel weniger als 10 Gew.-%, bevorzugt weniger als 5 Gew.-%, mehr bevorzugt weniger als 1 Gew.-%, jeweils bezogen auf das Gesamtgewicht des Reduktionsmittels, von Sulfonaten und von Sulfonat-Salzen verschiedenen Schwefel-Verbindungen, insbesondere weniger als 10 Gew.-%, besonders bevorzugt weniger als 5 Gew.-%, noch mehr bevorzugt weniger als 1 Gew.-% Sulfite, Bisulfite, Sulfinate oder Salze dieser Schwefelverbindungen beinhaltet.

Gemäß einer bevorzugten Ausführungsform der erfindungsgemäßen, wasserabsorbierenden Polymergebilde oder der durch das erfindungsgemäße Verfahren erhältlichen, wasserabsorbierenden Polymergebilde weisen diese mindestens eine der folgenden Eigenschaften auf:

(β1) einen gemäß der hierin beschriebenen Testmethode bestimmten Weiße-Index von mindestens 7,5, besonders bevorzugt von mindestens 8,0, noch mehr bevorzugt von mindestens 8,5 und am meisten bevorzugt von mindestens 9,0 nach einer Lagerung des wasserabsorbierenden Polymergebildes für 20 Tage bei 60°C und bei 75 % relativer Luftfeuchtigkeit;

(β2) eine nach ERT 442.2-02 bestimmte Absorption unter einem Druck von etwa 50 g/cm$^2$ (0,7 psi) von mindestens 18 g/g, besonders bevorzugt von mindestens 20 g/g und am meisten bevorzugt von mindestens 22 g/g;

(β3) bei einem gemäß der hierin beschriebenen Testmethode bestimmten TB-Wert < 26 g/g einen gemäß der hierin beschriebenen Testmethode bestimmten SFC-Wert (SFC = "*Saline Flow Conductivity*") von mindestens $80 \times 10^{-7}$ $cm^3 \cdot s \cdot g^{-1}$, bevorzugt von mindestens $100 \times 10^{-7}$ $cm^3 \cdot s \cdot g^{-1}$ und besonders bevorzugt von mindestens $120 \times 10^{-7}$ $cm^3 \cdot s \cdot g^{-1}$,

(β4) bei einem gemäß der hierin beschriebenen Testmethode bestimmten TB-Wert im Bereich ≥ 26 bis < 27 g/g einen gemäß der hierin beschriebenen Testmethode bestimmten SFC-Wert von mindestens $70 \times 10^{-7}$ $cm^3 \cdot s \cdot g^{-1}$, bevorzugt von mindestens $90 \times 10^{-7}$ $cm^3 \cdot s \cdot g^{-1}$ und besonders bevorzugt von mindestens $110 \times 10^{-7}$ $cm^3 \cdot s \cdot g^{-1}$,

(β5) bei einem gemäß der hierin beschriebenen Testmethode bestimmten TB-Wert im Bereich ≥ 27 bis < 28 g/g einen gemäß der hierin beschriebenen Testmethode bestimmten SFC-Wert von mindestens $60 \times 10^{-7}$ $cm^3 \cdot s \cdot g^{-1}$, bevorzugt von mindestens $80 \times 10^{-7}$ $cm^3 \cdot s \cdot g^{-1}$ und besonders bevorzugt von mindestens $100 \times 10^{-7}$ $cm^3 \cdot s \cdot g^{-1}$,

(β6) bei einem gemäß der hierin beschriebenen Testmethode bestimmten TB-Wert im Bereich ≥ 28 bis < 29 g/g einen gemäß der hierin beschriebenen Testmethode bestimmten SFC-Wert von mindestens $45 \times 10^{-7}$ $cm^3 \cdot s \cdot g^{-1}$, bevorzugt von mindestens $65 \times 10^{-7}$ $cm^3 \cdot s \cdot g^{-1}$und besonders bevorzugt von mindestens $85 \times 10^{-7}$ $cm^3 \cdot s \cdot g^{-1}$,

(β7) bei einem gemäß der hierin beschriebenen Testmethode bestimmten TB-Wert im Bereich ≥ 29 bis < 30 g/g einen gemäß der hierin beschriebenen Testmethode bestimmten SFC-Wert von mindestens $30 \times 10^{-7}$ $cm^3 \cdot s \cdot g^{-1}$, bevorzugt von mindestens $50 \times 10^{-7}$ $cm^3 \cdot s \cdot g^{-1}$ und besonders bevorzugt von mindestens $70 \times 10^{-7}$ $cm^3 \cdot s \cdot g^{-1}$,

(β8) bei einem gemäß der hierin beschriebenen Testmethode bestimmten TB-Wert im Bereich ≥30 bis < 31 einen gemäß der hierin beschriebenen Testmethode bestimmten SFC-Wert von mindestens $20 \times 10^{-7}$ $cm^3 \cdot s \cdot g^{-1}$, bevorzugt von mindestens $40 \times 10^{-7}$ $cm^3 \cdot s \cdot g^{-1}$ und besonders bevorzugt von mindestens $60 \times 10^{-7}$ $cm^3 \cdot s \cdot g^{-1}$,

(β9) bei einem gemäß der hierin beschriebenen Testmethode bestimmten TB-Wert im Bereich ≥ 31 g/g einen gemäß der hierin beschriebenen Testmethode bestimmten SFC-Wert von mindestens $10 \times 10^{-7}$ $cm^3 \cdot s \cdot g^{-1}$, bevorzugt von mindestens $20 \times 10^{-7}$ und besonders bevorzugt von mindestens $30 \times 10^{-7}$ $cm^3 \cdot s \cdot g^{-1}$.

Weiterhin bevorzugte Ausführungsformen der durch das erfindungsgemäße Verfahren erhältlichen wasserabsorbierenden Polymergebilde weisen jede denkbare Kombination der vorstehenden Merkmales (β1) bis (β9) auf, wobei die Ausführungsformen der folgenden Merkmalskombinationen bevorzugt sind: (β1), (β1)(β2), (β1)(β3), (β1)(β4), (β1)(β5), (β1)(β6), (β1)(β7), (β1)(β8), (β1)(β9) und (β1)(β2)(β3)(β4)(β5)(β6)(β7)(β8)(β9).

Dabei ist es bevorzugt, dass das erfindungsgemäße wasserabsorbierende Polymergebilde die gleichen Eigenschaften aufweist wie das durch das erfmdungsgemäße Verfahren erhältliche wasserabsorbierende Polymergebilde. Es ist auch erfindungsgemäß bevorzugt, dass diejenigen Werte, die im Zusammenhang mit dem erfindungsgemäßen Verfahren und den erfindungsgemäßen wasserabsorbierenden Polymergebilden als Untergrenzen von erfindungsgemäßen Merkmalen ohne Obergrenzen angegeben wurden, das 20-fache, vorzugsweise das 10-fache und besonders bevorzugt das 5-fache des am meisten bevorzugten Wertes der Untergrenze besitzen.

Weiterhin genannt wird ein Verbund, beinhaltend die erfindungsgemäßen wasserabsorbierenden Polymergebilde bzw. die durch das erfindungsgemäße Verfahren erhältlichen wasserabsorbierenden Polymergebilde und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher das erfindungsgemäße wasserabsorbierende Polymergebilde in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 $cm^3$, vorzugsweise mindestens 0,1 $cm^3$ und am meisten bevorzugt mindestens 0,5 $cm^3$ aufweist.

Bei dem Verbund handelt es sich insbesondere um einen flächenförmigen Verbund, wie er in der WO 02/056812 A1 als *"absorbent material"* beschrieben ist.

Es wird genannt ein Verfahren zur Herstellung eines Verbundes, wobei die erfindungsgemäßen wasserabsorbierenden Polymergebilde bzw. die durch das erfindungsgemäße Verfahren erhältlichen wasserabsorbierenden Polymergebilde und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Ferner wird genannt ein Verbund erhältlich nach dem vorstehend beschriebenen Verfahren, wobei dieser Verbund vorzugsweise die gleichen Eigenschaften aufweist wie der vorstehend beschriebene, Verbund.

Ferner werden genannt chemische Produkte beinhaltend die erfindungsgemäßen Polymergebilde oder einen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Polymergebilde oder des Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Die Erfindung wird nun anhand von Figuren, Testmethoden und nicht limitierenden Beispielen näher erläutert.

TESTMETHODEN

Bestimmung des SFC-Wertes und des TB-Wertes

Der SFC-Wert und der TB-Wert werden gemäß den in der DE-A-102 49 821 beschriebenen Testverfahren bestimmt.

Bestimmung des Weiße-Index

Der Weiße-Index wird über das L*,a*,b*-Farbsystem definiert. Der "L*"-Wert gibt die Helligkeit (100-0), der "a*"-Wert den Rotanteil (+) bzw. den Grün-Anteil (-) und der "b*"-Wert den Gelb-Anteil (+) bzw. den Blau-Anteil (-) an. Diese Skalierung basiert auf den Grundsätzen, welche in ASTM E 308 *"Standart Practice for Computing the Colors of Objects Using the CIE-System"* beschrieben sind.

Die Bestimmung der L*,a*,b*-Farbwerte erfolgt mittels eines Spektralkolorimeters *"Hunter LabScan XE"* (Hunter Associates Laboratory, Reston, VA, USA) bei folgenden Einstellungen:

| | |
|---|---|
| "Mode" | 0/45 |
| "Area View" | 44,5 mm |
| "Port Size" | 50,8 mm |
| "UV-Filter" | Nominal |

Vor jeder Messung wird der LabScan XE kalibriert, in dem zunächst die zum Gerätezubehör gehörende schwarze Glasplatte zwischen Probenteller und Messöffnung eingeklemmt wird, wobei die Glasplatte auf eine Petrischale (Durchmesser 100 mm, Tiefe: 15 mm) gelegt und durch Betätigung des "OK"-Schalters die Kalibrierung mit der schwarzen Glasplatte vollzogen wird. Anschließend wird auf die gleiche Art und Weise die weiße Standardplatte auf die Petrischale gelegt und erneut durch Betätigung des "OK"-Schalters die Kalibierung vollzogen.

Nachdem die Kalibierung durchgeführt wurde, wird der "Read Std"-Schalter gedrückt, um die Funktionsfähigkeit des Messgerätes zu prüfen, wobei dabei die Standardplatte noch nicht entfernt wird. Anschließend wird zur Messung der L*,a*,b*-Farbwerte für die Standardplatte der "Read"-Schalter betätigt.

Anschließend wird die Standardplatte entfernt und die Petrischale mit dem zu vermessenden wasserabsorbierenden Polymergebilde gefüllt, wobei die Produktoberfläche mit einem Abstreifer glattgezogen wird. Durch Drücken des "Read Sam"-Schalters wird die Probe vermessen.

$$\text{Der Weiße-Index ist definiert als } (L^*/b^*) - a^*$$

BEISPIELE

Herstellung des wasserabsorbierenden Polymergebildes

Eine Monomerlösung bestehend aus 640 g Acrylsäure, die zu 70 Mol-% mit Natronlauge neutralisiert wurde (497,36 g 50%ige NaOH), 825,06 g Wasser, 2,102 g Polyethylenglykol-300-diacrylat (76,1 %ig) und 4,010 g Polyethylenglykolmonoallylether (79,8 %ig, Molekulargewicht etwa 440 g/mol) wurde durch Spülen mit Stickstoff vom gelösten Sauerstoff befreit und auf die Starttemperatur von 4°C abgekühlt. Nach Erreichen der Starttemperatur wurde die Initiatorlösung (0,8 g Natriumperoxydisulfat in 10 g $H_2O$, 0,6 g 35 %ige Wasserstoffperoxid-Lösung in 10 g $H_2O$ und 0,06 g Ascorbinsäure in 10 g $H_2O$) zugesetzt. Nachdem die Endtempcratur von ca. 100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schlagkranzmühle (Firma Retsch ZM1) mit einem 5 mm-Sieb gemahlen und auf ein Pulver mit einer Partikelgröße von 150 bis 850 μm gesiebt.

Oberflächenmodifizierung

Das vorstehend erhaltene Polymerisat wurde mit einer wässrigen Lösung beinhaltend die in der nachfolgenden Tabelle 1 angegebenen Komponenten vermischt und für 30 Minuten bei 180°C erhitzt (die Gew.-%-Angaben beziehen sich auf die Menge an eingesetztem Polymerisat):

Tabelle 1

| | Vergleichsbeispiel | Beispiel 1 | Beispiel 2 | Beispiel 3 |
|---|---|---|---|---|
| Ethylencarbonat [Gew.-%] | 1,0 | 1,0 | 1,0 | 1,0 |
| Wasser [Gew.-%] | 3,0 | 3,0 | 3,0 | 3,0 |
| Aluminiumlactat [Gew.-%] | 0,4 | 0,4 | 0,4 | 0,4 |
| Aluminiumsulfat[1)] [Gew.-%] | 0,3 | 0,3 | 0,3 | 0,3 |
| Sulfonat[2)] [Gew.-%] | - | 0,15 | 0,2 | 0,25 |
| [1)] Eingesetzt als $Al_2(SO_4)_3 \times 14\ H_2O$<br>[2)] Das Dinatriumsalz der 2-Hydroxy-Sulfonatoessigsäure in reiner Form | | | | |

Von den so erhaltenen, wasserabsorbierenden Polymergebilden wurden die Permeabilität, die Retention und auch der Weiße-Index bestimmt. Die Ergebnisse sind der nachfolgenden Tabelle 2 zu entnehmen:

Tabelle 2

| | Vergleichsbeispiel | Beispiel 1 | Beispiel 2 | Beispiel 3 |
|---|---|---|---|---|
| SFC-Wert [$\times 10^{-7}\ cm^3 \cdot s/g$] | 75 | 93 | 73 | 73 |
| TB-Wert [g/g] | 29,3 | 29,7 | 29,9 | 30,4 |
| AAP-Wert [g/g] | 25,1 | 25,4 | 25,5 | 25,5 |
| Weiße-Index | 2,2 | 8,3 | 8,1 | 9,3 |

Der Tabelle 2 ist zu entnehmen, dass die erfindungsgemäßen Polymerisate im Vergleich zu herkömmlichen Polymerisaten einen deutlich verbesserten Weiße-Index bei vergleichbaren Absorptionseigenschaften aufweisen. Bei den in den Beispielen 1 bis 3 erhaltenen Polymerisaten konnten auch nach 20 tägiger Lagerung bei 60°C und bei 75 %iger, relativer Luftfeuchtigkeit keinerlei unangenehme Gerüche wahrgenommen werden.

**Patentansprüche**

**1.** Verfahren zur Herstellung wasserabsorbierender Polymergebilde, umfassend die Verfahrensschritte:

i) Bereitstellen einer wässrigen Monomerlösung beinhaltend

- ein polymerisierbares, monoethylenisch ungesättigtes, eine Säuregruppetragendes Monomer ($\alpha$1) oder ein Salz davon,
- gegebenenfalls ein mit dem Monomer ($\alpha$1) polymerisierbares, monoethylenisch ungesättigtes Monomer ($\alpha$2), sowie
- gegebenenfalls einen Vernetzer ($\alpha$3),

ii) radikalische Polymerisation der wässrigen Monomerlösung unter Erhalt eines Polymergels,
iii) gegebenenfalls Zerkleinern des Polymergels,
iv) Trocknen des gegebenenfalls zerkleinerten Polymergels unter Erhalt wasserabsorbierender Polymergebilde,
v) gegebenenfalls Mahlen und Absieben der wasserabsorbierenden Polymergebilde, sowie
vi) Oberflächennachvernetzung der gegebenenfalls gemahlenen und abgesiebten wasserabsorbierenden Polymergebilde,

wobei

I) dem wasserabsorbierenden Polymergebilde nach Durchführung des Verfahrensschrittes iv),
II) dem wasserabsorbierenden Polymergebilde nach Durchführung des Verfahrensschrittes v), oder
III) dem wasserabsorbierenden Polymergebilde nach Durchführung des Verfahrensschrittes vi),

ein Reduktionsmittel, beinhaltend ein Sulfonat, ein Salz eines Sulfonates oder eine Mischung aus einem Sulfonat und einem Salz eines Sulfonates, zugesetzt wird, wobei es sich um 2-Hydroxy-2-Sulfonate-essigsäure, insbesondere deren Dinatriumsalz handelt,
wobei das Reduktionsmittel weniger als 10 Gew.-%, bezogen auf das Gesamtgewicht des Reduktionsmittels, an von Sulfonaten verschiedenen Schwefel-Verbindungen beinhaltet.

**2.** Das Verfahren nach Anspruch 1, wobei das Salz des Sulfonates das Dinatrium-Salz der 2-Hydroxy-2-Sulfonatoessigsäure ist.

**3.** Das Verfahren nach einem der vorhergehenden Ansprüche 1 oder 2, wobei das Reduktionsmittel in einer Menge in einem Bereich von 0,001 bis 10 Gew.-%, bezogen auf den Feststoffgehalt des wasserabsorbierenden Polymergebildes, eingesetzt wird.

**4.** Das Verfahren nach einem der vorhergehenden Ansprüche, wobei das Reduktionsmittel in Form eines Fluids beinhaltend das Reduktionsmittel sowie ein Lösungsmittel zugesetzt wird.

**5.** Das Verfahren nach Anspruch 4, wobei das Lösungsmittel Wasser ist.

**6.** Wasserabsorbierendes Polymergebilde, erhältlich durch ein Verfahren nach einem der vorhergehenden Ansprüche.

**7.** Wasserabsorbierendes Polymergebilde, beinhaltend 10 bis 100.000 ppm, bezogen auf den Feststoffgehalt des wasserabsorbierenden Polymergebildes, eines nicht polymerisierten Sulfonates, eines nicht polymerisierten Salzes eines Sulfonates oder einer Mischung aus einem nicht polymerisierten Sulfonat und einem nicht polymerisierten Salz eines Sulfonates, sowie weniger als 1.000 ppm, bezogen auf den Feststoffgehalt des wasserabsorbierenden Polymergebildes, nicht polymerisierte Sulfite, nicht polymerisierte Bisulfite, nicht polymerisierte Sulfinate oder nicht polymerisierte Salze dieser Verbindungen,
wobei das Sulfonat bzw. das Salz des Sulfonates 2-Hydroxy-2-Sulfonato-essigsäure, insbesondere das Salz des Sulfonates das Dinatrium-Salz der 2-Hydroxy-2-Sulfonatoessigsäure ist.

**8.** Wasserabsorbierendes Polymergebilde, dessen Oberfläche mit 0,001 bis 10 Gew.-%, bezogen auf den Feststoffgehalt des wasserabsorbierenden Polymergebildes, eines Reduktionsmittels beinhaltend ein Sulfonat, ein Salz eines Sulfonates oder eine Mischung aus einem Sulfonat und einem Salz eines Sulfonates in Kontakt gebracht worden ist, wobei das Reduktionsmittel weniger als 10 Gew.-%, bezogen auf das Gesamtgewicht des Reduktionsmittels, an von Sulfonaten verschiedenen Schwefel-Verbindungen beinhaltet,
wobei das Sulfonat bzw. das Salz des Sulfonates, 2-Hydroxy-2-Sulfonato-essigsäure, insbesondere das Salz des Sulfonates das Dinatrium-Salz der 2-Hydroxy-2-Sulfonatoessigsäure ist.

**9.** Das wasserabsorbierende Polymergebilde nach Anspruch 8, wobei das Reduktionsmittel weniger als 10 Gew.-%, bezogen auf das Gesamtgewicht des Reduktionsmittels, Sulfite, Bisulfite, Sulfinate oder Salze dieser Schwefelverbindungen beinhaltet.

**10.** Das wasserabsorbierende Polymergebilde nach einem der Ansprüche 6 bis 11, mindestens eine der folgenden Eigenschaften aufweisend:

($\beta$1) einen gemäß der hierin beschriebenen Testmethode bestimmten Weiße-Index von mindestens 7,5 nach einer Lagerung des wasserabsorbierenden Polymergebildes für 20 Tage bei 60°C und bei 75 % relativer Luftfeuchtigkeit;
($\beta$2) eine nach ERT 442.2-02 bestimmte Absorption unter einem Druck von etwa 50 g/cm$^2$ (0,7 psi) von mindestens 18 g/g;
($\beta$3) bei einem gemäß der hierin beschriebenen Testmethode bestimmten TB-Wert < 26 g/g einen gemäß der hierin beschriebenen Testmethode bestimmten SFC-Wert (SFC = "*Saline Flow Conductivity*") von mindestens 80x $10^{-7}$ $cm^3 \cdot s \cdot g^{-1}$,
($\beta$4) bei einem gemäß der hierin beschriebenen Testmethode bestimmten TB-Wert im Bereich ≥ 26 bis < 27 g/g einen gemäß der hierin beschriebenen Testmethode bestimmten SFC-Wert von mindestens 70 × $10^{-7}$ $cm^3 \cdot s \cdot g^{-1}$, bevorzugt von mindestens 90 × $10^{-7}$ $cm^3 \cdot s \cdot g^{-1}$,
($\beta$5) bei einem gemäß der hierin beschriebenen Testmethode bestimmten TB-Wert im Bereich ≥ 27 bis < 28 g/g einen gemäß der hierin beschriebenen Testmethode bestimmten SFC-Wert von mindestens 60 × $10^{-7}$ $cm^3 \cdot s \cdot g^{-1}$,

(β6) bei einem gemäß der hierin beschriebenen Testmethode bestimmten TB-Wert im Bereich ≥ 28 bis < 29 g/g einen gemäß der hierin beschriebenen Testmethode bestimmten SFC-Wert von mindestens 45 × $10^{-7}$ $cm^3 \cdot s \cdot g^{-1}$,
(β7) bei einem gemäß der hierin beschriebenen Testmethode bestimmten TB-Wert im Bereich ≥ 29 bis < 30 g/g einen gemäß der hierin beschriebenen Testmethode bestimmten SFC-Wert von mindestens 30 × $10^{-7}$ $cm^3 \cdot s \cdot g^{-1}$,
(β8) bei einem gemäß der hierin beschriebenen Testmethode bestimmten TB-Wert im Bereich ≥30 bis < 31 g/g einen gemäß der hierin beschriebenen Testmethode bestimmten SFC-Wert von mindestens 20 × $10^{-7}$ $cm^3 \cdot s \cdot g^{-1}$,
(β9) bei einem gemäß der hierin beschriebenen Testmethode bestimmten TB-Wert im Bereich ≥ 31 g/g einen gemäß der hierin beschriebenen Testmethode bestimmten SFC-Wert von mindestens 10 × $10^{-7}$ $cm^3 \cdot s \cdot g^{-1}$.

**11.** Verwendung des wasserabsorbierenden Polymergebilde nach einem der Ansprüche 6 bis 10 in Schäumen, Formkörpern, Fasern, Folien, Filmen, Kabeln, Dichtungsmaterialien, flüssigkeitsaufnehmenden Hygieneartikeln, Trägem für pflanzen- und pilzwachstumregulierenden Mitteln, Verpackungsmaterialien, Bodenzusätzen, zur kontrollierten Freisetzung von Wirkstoffen oder in Baustoffen.

## Claims

**1.** A process for the production of water-absorbing polymer structures, comprising the process steps:

i) providing an aqueous monomer solution comprising

- a polymerizable, monoethylenically unsaturated monomer (α1) carrying an acid group or a salt thereof,
- optionally a monoethylenically unsaturated monomer (α2) which can be polymerized with the monomer (α1), and
- optionally a crosslinking agent (α3),

ii) free-radically polymerizing the aqueous monomer solution to give a polymer gel,
iii) optionally comminuting the polymer gel,
iv) drying the optionally comminuted polymer gel to give water-absorbing polymer structures,
v) optionally grinding and sieving the water-absorbing polymer structures, and
vi) surface postcrosslinking the optionally ground and sieved water-absorbing polymer structures,

wherein
a reducing agent comprising a sulphonate, a salt of a sulphonate or a mixture of a sulphonate and a salt of a sulphonate, wherein 2-hydroxy-2-sulphonatoacetic acid, especially its disodium salt, is concerned, is added to

I) the water-absorbing polymer structure after carrying out process step iv),
II) the water-absorbing polymer structure after carrying out process step v), or
III) the water-absorbing polymer structure after carrying out process step vi), wherein the reducing agent comprises less than 10 wt.% of sulphur compounds other than sulphonates, based on the total weight of the reducing agent.

**2.** The process according to claim 1 wherein the salt of the sulphonate is the disodium salt of 2-hydroxy-2-sulphonatoacetic acid.

**3.** The process according to either preceding claim 1 or 2 wherein the reducing agent is employed in an amount in a range of from 0.001 to 10 wt.%, based on the solids content of the water-absorbing polymer structure.

**4.** The process according to any preceding claim wherein the reducing agent is added in the form of a fluid comprising the reducing agent and a solvent.

**5.** The process according to claim 4 wherein the solvent is water.

**6.** A water-absorbing polymer structure obtainable by a process according to any preceding claim.

**7.** A water-absorbing polymer structure comprising 10 to 100 000 ppm, based on the solids content of the water-absorbing polymer structure, of a non-polymerized sulphonate, a non-polymerized salt of a sulphonate or a mixture of a non-polymerized sulphonate and a non-polymerized salt of a sulphonate, and less than 1000 ppm, based on the solids content of the water-absorbing polymer structure, of non-polymerized sulphites, non-polymerized bisulphites, non-polymerized sulphinates or non-polymerized salts of these compounds, wherein the sulphonate or the salt of the sulphonate is 2-hydroxy-2-sulphonatoacetic acid, especially the salt of the sulphonate is the disodium salt of 2-hydroxy-2-sulphonatoacetic acid.

**8.** A water-absorbing polymer structure, the surface of which has been brought into contact with 0.001 to 10 wt.%, based on the solids content of the water-absorbing polymer structure, of a reducing agent comprising a sulphonate, a salt of a sulphonate or a mixture of a sulphonate and a salt of a sulphonate, wherein the reducing agent comprises less than 10 wt.%, based on the total weight of the reducing agent, of sulphur compounds other than sulphonates, wherein the sulphonate or the salt of the sulphonate is 2-hydroxy-2-sulphonatoacetic acid, especially the salt of the sulphonate is the disodium salt of 2-hydroxy-2-sulphonatoacetic acid.

**9.** The water-absorbing polymer structure according to claim 8 wherein the reducing agent comprises less than 10 wt. %, based on the total weight of the reducing agent, of sulphites, bisulphites, sulphinates or salts of these sulphur compounds.

**10.** The water-absorbing polymer structure according to any of claims 6 to 9 having at least one of the following properties:

(β1) a whiteness index, determined in accordance with the test method described herein, of at least 7.5 after storage of the water-absorbing polymer structure for 20 days at 60°C and at 75% relative atmospheric humidity;
(β2) an absorption, determined in accordance with ERT 442.2-02, under a pressure of about 50 g/cm$^2$ (0.7 psi) of at least 18 g/g;
(β3) an SFC value (SFC = "Saline Flow Conductivity"), determined in accordance with the test method described herein, at a TB value of < 26 g/g, determined in accordance with the test method described herein, of at least $80 \times 10^{-7}$ cm$^3$·s·g$^{-1}$,
(β4) an SFC value, determined in accordance with the test method described herein, at a TB value of ≥ 26 to < 27 g/g, determined in accordance with the test method described herein, of at least $70 \times 10^{-7}$ cm$^3$·s·g$^{-1}$, preferably of at least $90 \times 10^{-7}$ cm$^3$·s·g$^{-1}$,
(β5) an SFC value, determined in accordance with the test method described herein, at a TB value of ≥ 27 to < 28 g/g, determined in accordance with the test method described herein, of at least $60 \times 10^{-7}$ cm$^3$·s·g$^{-1}$,
(β6) an SFC value, determined in accordance with the test method described herein, at a TB value of ≥ 28 to < 29 g/g, determined in accordance with the test method described herein, of at least $45 \times 10^{-7}$ cm$^3$·s·g$^{-1}$,
(β7) an SFC value, determined in accordance with the test method described herein, at a TB value of ≥ 29 to < 30 g/g, determined in accordance with the test method described herein, of at least $30 \times 10^{-7}$ cm$^3$·s·g$^{-1}$,
(β8) an SFC value, determined in accordance with the test method described herein, at a TB value of ≥ 30 to < 31 g/g, determined in accordance with the test method described herein, of at least $20 \times 10^{-7}$ cm$^3$·s·g$^{-1}$,
(β9) an SFC value, determined in accordance with the test method described herein, at a TB value of ≥ 31 g/g, determined in accordance with the test method described herein, of at least $10 \times 10^{-7}$ cm$^3$·s·g$^{-1}$.

**11.** The use of the water-absorbing polymer structure according to any of claims 6 to 10 in foams, shaped articles, fibres, foils, films, cables, sealing materials, liquid-absorbing hygiene articles, carriers for plant and fungal growth-regulating agents, packaging materials, soil additives, for controlled release of active compounds or in building materials.

**Revendications**

**1.** Procédé de fabrication de structures polymères absorbant l'eau, comprenant les étapes de procédé suivantes :

i) la préparation d'une solution aqueuse de monomères contenant :

- un monomère (α1) polymérisable, monoéthyléniquement insaturé, portant un groupe acide, ou un sel de celui-ci,
- éventuellement un monomère (α2) monoéthyléniquement insaturé, polymérisable avec le monomère (α1), et
- éventuellement un agent de réticulation (α3),

ii) la polymérisation radicalaire de la solution aqueuse de monomères pour obtenir un gel polymère,
iii) éventuellement le broyage du gel polymère,
iv) le séchage du gel polymère éventuellement broyé pour obtenir des structures polymères absorbant l'eau,
v) éventuellement le broyage et le tamisage des structures polymères absorbant l'eau, et
vi) la post-réticulation de surface des structures polymères absorbant l'eau éventuellement broyées et tamisées,

un réducteur, contenant un sulfonate, un sel d'un sulfonate ou un mélange d'un sulfonate et d'un sel d'un sulfonate, celui-ci étant l'acide 2-hydroxy-2-sulfonato-acétique, notamment son sel disodique, étant ajouté

I) à la structure polymère absorbant l'eau après la réalisation de l'étape de procédé iv),
II) à la structure polymère absorbant l'eau après la réalisation de l'étape de procédé v),
ou
III) à la structure polymère absorbant l'eau après la réalisation de l'étape de procédé vi),
le réducteur contenant moins de 10 % en poids, par rapport au poids total du réducteur, de composés de soufre différents de sulfonates.

**2.** Procédé selon la revendication 1, dans lequel le sel du sulfonate est le sel disodique de l'acide 2-hydroxy-2-sulfonato-acétique.

**3.** Procédé selon l'une quelconque des revendications 1 ou 2 précédentes, dans lequel le réducteur est utilisé en une quantité dans une plage allant de 0,001 à 10 % en poids, par rapport à la teneur en solides de la structure polymère absorbant l'eau.

**4.** Procédé selon l'une quelconque des revendications précédentes, dans lequel le réducteur est ajouté sous la forme d'un fluide contenant le réducteur et un solvant.

**5.** Procédé selon la revendication 4, dans lequel le solvant est l'eau.

**6.** Structure polymère absorbant l'eau, pouvant être obtenue par un procédé selon l'une quelconque des revendications précédentes.

**7.** Structure polymère absorbant l'eau, contenant 10 à 100 000 ppm, par rapport à la teneur en solides de la structure polymère absorbant l'eau, d'un sulfonate non polymérisé, d'un sel d'un sulfonate non polymérisé ou d'un mélange d'un sulfonate non polymérisé et d'un sel d'un sulfonate non polymérisé, ainsi que moins de 1 000 ppm, par rapport à la teneur en solides de la structure polymère absorbant l'eau, de sulfites non polymérisés, de bisulfites non polymérisés, de sulfinates non polymérisés ou de sels de ces composés non polymérisés,
le sulfonate ou le sel du sulfonate étant l'acide 2-hydroxy-2-sulfonato-acétique, le sel du sulfonate étant notamment le sel disodique de l'acide 2-hydroxy-2-sulfonato-acétique.

**8.** Structure polymère absorbant l'eau, dont la surface a été mise en contact avec 0,001 à 10 % en poids, par rapport à la teneur en solides de la structure polymère absorbant l'eau, d'un réducteur contenant un sulfonate, un sel d'un sulfonate ou un mélange d'un sulfonate et d'un sel d'un sulfonate, le réducteur contenant moins de 10 % en poids, par rapport au poids total du réducteur, de composés de soufre différents de sulfonates,
le sulfonate ou le sel du sulfonate étant l'acide 2-hydroxy-2-sulfonato-acétique, le sel du sulfonate étant notamment le sel disodique de l'acide 2-hydroxy-2-sulfonato-acétique.

**9.** Structure polymère absorbant l'eau selon la revendication 8, dans laquelle le réducteur contient moins de 10 % en poids, par rapport au poids total du réducteur, de sulfites, de bisulfites, de sulfinates ou de sels de ces composés de soufre.

**10.** Structure polymère absorbant l'eau selon l'une quelconque des revendications 6 à 9, présentant au moins l'une des propriétés suivantes :

($\beta$1) un indice de blancheur déterminé selon la méthode de test décrite dans le présent document d'au moins 7,5 après un stockage de la structure polymère absorbant l'eau pendant 20 jours à 60 °C et 75 % d'humidité relative de l'air ;
($\beta$2) une absorption sous une pression d'environ 50 g/$cm^2$ (0,7 psi) déterminée selon ERT 442.2-02 d'au moins 18 g/g ;

(β3) à une valeur TB déterminée selon la méthode de test décrite dans le présent document < 26 g/g, une valeur SFC (SFC = « Saline Flow Conductivity ») déterminée selon la méthode de test décrite dans le présent document d'au moins 80 x $10^{-7}$ $cm^3 \cdot s \cdot g^{-1}$,
(β4) à une valeur TB déterminée selon la méthode de test décrite dans le présent document dans la plage allant de ≥ 26 à < 27 g/g, une valeur SFC déterminée selon la méthode de test décrite dans le présent document d'au moins 70 x $10^{-7}$ $cm^3 \cdot s \cdot g^{-1}$, de préférence d'au moins 90 x $10^{-7}$ $cm^3 \cdot s \cdot g^{-1}$,
(β5) à une valeur TB déterminée selon la méthode de test décrite dans le présent document dans la plage allant de ≥ 27 à < 28 g/g, une valeur SFC déterminée selon la méthode de test décrite dans le présent document d'au moins 60 x $10^{-7}$ $cm^3 \cdot s \cdot g^{-1}$,
(β6) à une valeur TB déterminée selon la méthode de test décrite dans le présent document dans la plage allant de ≥ 28 à < 29 g/g, une valeur SFC déterminée selon la méthode de test décrite dans le présent document d'au moins 45 x $10^{-7}$ $cm^3 \cdot s \cdot g^{-1}$,
(β7) à une valeur TB déterminée selon la méthode de test décrite dans le présent document dans la plage allant de ≥ 29 à < 30 g/g, une valeur SFC déterminée selon la méthode de test décrite dans le présent document d'au moins 30 x $10^{-7}$ $cm^3 \cdot s \cdot g^{-1}$,
(β8) à une valeur TB déterminée selon la méthode de test décrite dans le présent document dans la plage allant de ≥ 30 à < 31 g/g, une valeur SFC déterminée selon la méthode de test décrite dans le présent document d'au moins 20 x $10^{-7}$ $cm^3 \cdot s \cdot g^{-1}$,
(β9) à une valeur TB déterminée selon la méthode de test décrite dans le présent document dans la plage ≥ 31 g/g, une valeur SFC déterminée selon la méthode de test décrite dans le présent document d'au moins 10 x $10^{-7}$ $cm^3 \cdot s \cdot g^{-1}$.

**11.** Utilisation de la structure polymère absorbant l'eau selon l'une quelconque des revendications 6 à 10 dans des mousses, des corps moulés, des fibres, des feuilles, des films, des câbles, des matériaux d'étanchéité, des articles d'hygiène absorbant les liquides, des supports pour agents de régulation de la croissance des plantes et des champignons, des matériaux d'emballage, des additifs de sol, pour la libération contrôlée d'agents actifs ou dans des matériaux de construction.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004084962 A
- DE 19529348 A1
- WO 9934843 A1
- WO 2004037903 A2
- US 4286082 A
- DE 2706135 A1
- US 4076663 A
- DE 3503458 A1
- DE 4020780 C1
- DE 4244548 A1
- DE 4333056 A1
- DE 4418818 A1
- US 4340706 A
- DE 3713601 A1
- DE 2840010 A1
- WO 9605234 A1
- DE 10334286 A
- WO 02056812 A1
- DE 10249821 A


### In der Beschreibung aufgeführte Nicht-Patentliteratur


- **F. L. BUCHHOLZ ; A. T. GRAHAM.** Modern Superabsorbent Polymer Technology. Wiley-VCH, 1998